# EUROPEAN PATENT SPECIFICATION

(11) **EP 0 306 256 B1**
(45) Date of publication and mention of the grant of the patent: **09.03.1994**
(21) Application number: 88308000.4
(22) Date of filing: 30.08.1988
(51) Int. Cl.: A61L 27/00, A61F 2/04

(54) **Bioprosthetic valve**
Bioprosthetisches Ventil
Valve bioprosthétique

(30) Priority: 31.08.1987 JP 215365/87
(43) Date of publication of application: 08.03.1989
(73) Proprietor: KOKEN CO. LTD., Shinjuku-ku Tokyo (JP)
(72) Inventor: Imamura, Eisaburo, Tokyo, 186 (JP); Noishiki, Yasuharu, Tohaku-gun Tottori-ken, 682-02 (JP); Koyanagi, Hitoshi, Bunkyo-ku Tokyo 113 (JP); Miyata, Teruo, Shinjuku-ku Tokyo, 161 (JP); Furuse, Masayasu, Kanagawa-ken, 228 (JP)
(74) Representative: Gore, Peter Manson

(56) References cited:
- EP-A- 0 212 933
- PATENT ABSTRACTS OF JAPAN, unexamined applications, C section, vol. 11, no. 202, June 30, 1987 THE PATENT OFFICE JAPANESE GOVERNMENT page 89 C 432
- THE JOURNAL OF THORACIC AND CARDIOVASCULAR SURGERY, vol. 86, (1983) pp. 115-125
- THE AMERICAN JOURNAL OF CARDIOLOGY, vol. 46, Nov. 5, 1980, pp. 721-734
- CARDIOLOGY CLINICS, vol. 3, no. 3, Aug. 1985, pp. 385-396

## Description

The present invention relates to a bioprosthetic valve for use in valve replacement and in artificial hearts. More particularly, it relates to a bioprosthetic valve which is not calcified and has excellent biocompatibility.

From the middle of the 1950's valve replacement operations have been practised. A wide variety of artifical valves have been developed and now they are widely used in replacement for valves such as, for example, aortic valves, mitral valves and tricuspid valves, and also in artificial hearts.

Artificial valves are classified broadly into mechanoprosthetic valves and bioprosthetic valves. Mechanoprosthetic valves are further classified generally into ball-type valves and disc-type valves, depending on the shape of moving portions. However, both types of the mechanoprosthetic valves have the following disadvantages: (1) there is not parallel flow through the bloodstream, used therewith; (2) the materials which are used to make the valves readily form thrombus and deteriorate blood components to cause haemolysis, and (3) the sounds made by the valves have an adverse psychological effect on the patient. Bioprosthetic valves now clinically used include porcine aortic valves treated with glutaraldehyde (hereinafter referred to as GA) and bovine pericardia formed into the form of a valve cusp. Such valves have an excellent antithrombogenic property. This eliminates the use of anticoagulants (such as, for example, Warfarin®) which can possibly cause cerebral haemorrhaging. Such valves also have haemodynamic properties which provide a central flow characteristic. However, they have problems in durability in that the physical properties of the valve are such that calcium deposits on the valve cusp tissues, and cracks and perforations, are caused by the fatigue of the valves.

EP-A-212933 discloses a method of preparing an antithrombogenic medical material with improved antithrombogenic properties as well as improved physical characteristics, particularly flexibility, which comprises the steps of fixing a protamine to a collagen or derivative thereof through a polyepoxy compound and heporising the collagen by fixing herporin to the protamine.

Valves using biological tissues are crosslinked with GA in order to maintain their strength, to suppress their being absorbed into living bodies, and to reduce their antigenic property. However, the crosslinking with GA treatment inevitably makes the biological tissues stiff. The stiffened valve cusps behave differently from untreated, natural valves. Abnormal behaviour of the stiffened valve cusps becomes more significant at lower blood pressures.

About half of GA treated valves can become dysfunctional owing to calcification and cracks within 5 to 10 years after their implantation into human bodies due to calcification and cracking. In particular, with young children showing vigorous calcium metabolism, almost all the GA treated valves implanted are subject to calcification and become dysfunctional even earlier after the Implantation (may be 2 to 6 years). Thus, GA has various disadvantages as a crosslinking agent for biological tissue to be used as a valvve in the bodies of humans and mammals.

The following requirements for a crosslinking agent for crosslinking biological tissue would provide an ideal bioprosthetic valve:
(1) completely excludes the antigenecity of heterologus animal tissues;
(2) prevents the invasion of calcium into biological tissues;
(3) has persistent sterilizing effects and strong resistance to infections;
(4) is free from deterioration of flexibility and elasticity of biological tissues and allows valves to behave similarly to natural valves; and
(5) provides valves with excellent tensile strength and torsional strength.

Ideal crosslinking agents which can substituted for GA and satisfy the above noted requirements have long been desired.

The inventors of the present Application have investigated various crosslinking agents satisfying the above-noted requirements, and have found that polyepoxy compounds have satisfactory properties and do not easily cause calcification.

According to the present invention there is provided a bioprosthetic valve comprising a biological tissue derived from a mammal containing crosslinked collagen in which the crosslinked collagen consists essentially of collagen which has been crosslinked with a polyepoxy compound, wherein the polyepoxy compound is selected from glycerol diglycidyl ether, diglycerol triglycidyl ether, diglycerol tetraglycidyl ether and ethylene glycol glycidyl ether.

As biological tissue to be crosslinked according to the present invention, tissues of various mammals such as, for example, pig, cattle, sheep, cow and goat, may be used. When such tissues are used as they are, they may be valve tissues such as, for example, aortic valves, pulmonary valves, vena caval valves, mitral valves and tricupsid valves. When valve cusps of bioprosthetic valves of the present invention are produced, they may be formed of membrane-like tissues. The membrane-like tissues may be, for example, pericardium, dura mater, amnion and fascia.

The calcifying mechanism of biological tissues of heterologus animals is not completely understood at this time. It is therefore difficult to explain theoretically why no calcification occurred on the valves treated with the polyepoxy compounds in accordance with the present invention and, in contrast, the GA treated valves were rapidly subject to heavy calcification.

However, crosslinked tissues with polyepoxy compounds are hydrophilic in contrast to tissues cross-linked with GA, which are hydrophobic. The crosslinking with polyepoxy compounds maintains flexibility and elasticity of the tissues and the valves crosslinked therewith behave similarly to normal, natural valves. In contrast, crosslinking with GA results in valves which have lost elasticity of biological tissues. This difference results in excellent antithrombogenic properties being obtained for tissues crosslinked with the polyepoxy compounds as compared to crosslinking with GA.

It is well known that calcification occurs on thrombi. Calcification of bioprosthetic valves has been observed often in the portions of valves where more stress is applied. By crosslinking by glutaraldehyde, in particular, the biological tissue used for the valve is stiffened, to the extent that, when the valve moves in a manner to cause frequent bending, such a stiffened valve has sharply bent portions. In such case, repeated bending of the bent portion causes small cracks to occur. The collagen exposed by the cracks will induce thrombi, or calcium ions will pass through openings of the cracks to cause calcification.

Attention has been given to the fact that polyepoxy compounds which are generally used in the textile industry are highly reactive to an amino group and a carboxylic group. Collagen crosslinked with the polyepoxy compounds is excellent in biocompatibility and has flexibility close to that of biological tissues, as disclosed in Japanese Patent Application Laid-Open No.26230/87 entitled "Crosslinked Medical Material".

The amount or percentage of the ε-amino group on the side chains of collagen which react during crosslinking can be taken as the criterion of the amount of crosslinking. According to the present invention, it has been discovered that, as compared with GA treatment, the percentage of ε-amino group which react is in the range of from 5 to 90%, preferably from 10 to 60%, depending on the polyepoxy compound to be used and the biological tissue to be crosslinked. This results in a crosslinked collagen having excellent physical properties such as, for example, strength and elongation. Thus, an amount of crosslinking within the range of 5 to 90%, preferably 10 to 60%, of the collagen with the polyepoxy compound can provide a bioprosthetic valve having excellent biocompatability, flexibility and resistance to calcification.

In addition, it can be expected that the collagen crosslinked with a polyepoxy compound in accordance with the present invention will be considerably stable in living bodies because the crosslinked collagen has a solubility of 30% in the digestion trial with bacterial collagenase, although the solubility is a little higher than that of GA treated collagen.

The blood vessels of a dog, which had been crosslinked with GA and a glycerol polyglycidyl ether (GPGE), respectively, were sensitized in mice to determine antibodies with the result of no detection thereof. In addition, the blood vessel crosslinked with GPGE had an antigenic property reduced as low as that of one crosslinked with GA.

Further, the porcine aortic valves, which had been crosslinked with GPGE and GA, respectively, were subcutaneously implanted into infant rats to measure the amount of calcium deposits. The amount of calcium deposits when compared to that of a fresh, untreated valve (control) was more than 200 times only one month after the implantation in the case of the GA crosslinking; in contrast, the amount was about two times even three months after implantation in the case of the GPGE crosslinked valve. Thus the bioprosphetic valves comprising the biological tissues crosslinked with the polyepoxy compounds have been proved to have excellent biocompatibility and physical properties, and durability, and are not subject to calcification.

The bioprosthetic valves obtained by the crosslinking treatment with the polyepoxy compounds have a potential value of application as a valve substitute of an auxiliary heart which has an absolute requirement of antithrombogenic property and durability.

The present invention will now be described in detail with reference to the following Examples which are not construed as limiting the invention in any way. In the Examples, Decanol EX-313® and Decanol EX-314® are used as the polyepoxy compounds. Decanol EX-313® and Decanol EX-314® (which are trade names of Nagase Kasie Kogyo K.K.) are respectively glycerol diglycidyl ether and glycerol triglycidyl ether. These compounds have the following structural formulae: 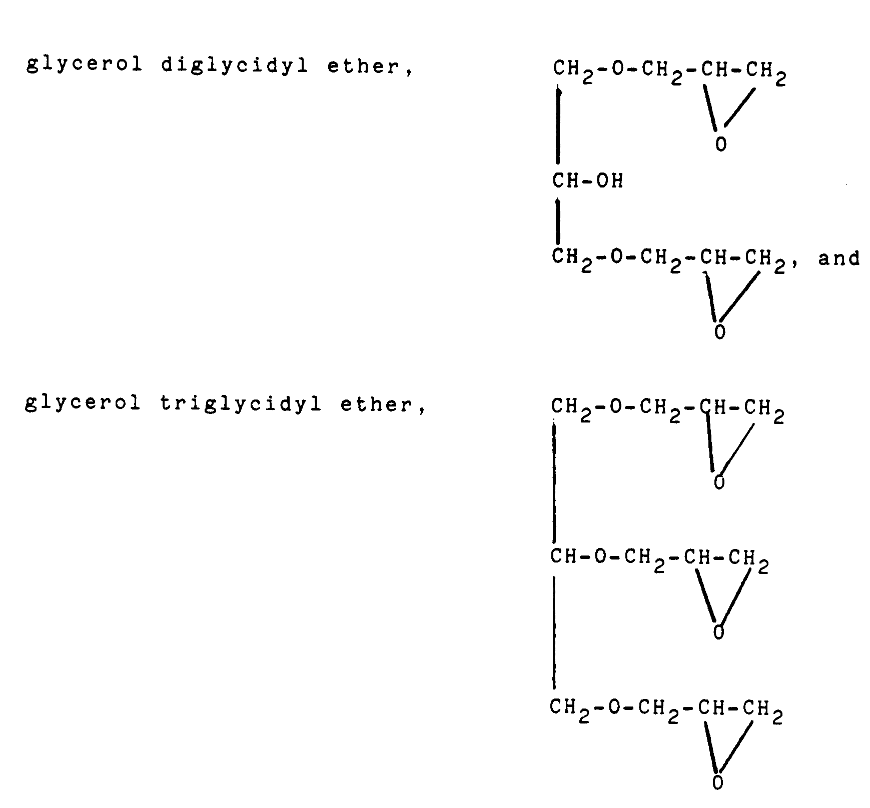
Of course, other polyepoxy compounds can be used.

### Example 1

A valve cusp was cut out of a fresh porcine aortic valve. The valve cusp was immersed in a carbonate buffer solution and then immersed in a 2% solution (pH9.0) of Denacol EX-314® in a phosphate buffer at 20°C for 48 hours. The valve cusp was then washed thoroughly with water and immersed in a saline solution. The valve cusp thus treated was used for implantation experiments to evaluate the calcification preventing effects of glycerol polyglycidil ether crosslinked animal tissue.

For comparison, a valve cusp tissue was immersed in a 0.625% solution (pH7.0) of glutaraldehyde in a phosphate buffer at 20°C for 48 hours. Then the valve cusp was washed thoroughly with water, immersed in a saline and then subjected to implantation experiments.

The following implantation experiments were conducted. The valve cusps treated as above were implanted into the subcutaneous tissue on the back of a four-weeks-old rat. The implants were recovered after one to three months and the amount of calcium deposits per unit dry weight was measured by atomic absorption spectrophotometry. The results are shown in the Table below. The glutaraldehyde-treated valves were subject to calcification in amounts as much as from 90 to 170 µg/mg (average 140 µg/mg) in the same period after implantation. In contrast thereto, the amounts of calcium deposits of the glycerol polyglycidyl ether-treated valves were below 1 µg per mg of dry tissue one to three months after implantation and the valves were subject to substantially no calcification as compared to 0.4µg/mg for the fresh, untreated valve cusp tissue.

**TABLE**

| Amount of Calcium Deposits On Valve Cusp Tissue of Porcine Aortic Valve in Subcutaneous Implantation Into Rat (ug/mg dry weight tissue)* | | | |
|---|---|---|---|
| Period After Implantation (month) | Untreated No-Implantation | Crosslinking Through | |
| | | Epoxy Group | Aldehyde Group |
| 1 | - | 0.64+0.05 (n=7) | 90.8+7.9 (n=11) |
| 2 | - | 0.94+0.06 (n=9) | 135.5+10.6 (n=16) |
| 3 | - | 0.96+0.15 (n=10) | 170.0+7.1 (n=21) |
| mean value | 0.43+0.05 | 0.96+0.07 (n=25) | 140.7+6.6 (n=47) |

| | | | |
|---|---|---|---|
| *Numerical valves are expressed as mean+standard error | | | |
| n = number of implantations removed | | | |

### Example 2

The pericardium of a cow was immersed in 0.01% aqueous ficin for 24 hours to remove proteins other than collagen, and the pericardium was then washed thoroughly with water. A 1mm diameter stainless steel wire was formed into a frame for a bioprosthetic valve. The periphery of the frame was covered with a polyester cloth and the pericardium treated as above was used to prepare a bioprosthetic valve. The resulting valve was allowed to react by immersing it in a solution containing 10g of Denacol Ex-313®, 0.1g of 2,3,6-tris (dimethylaminomethyl) phenol as a catalyst, 0.07g of salicylic acid as a reaction accelerator, and 10ml of ethanol dissolved in 80ml of 0.1 N NaOH at 20°C for 24 hours. The valve so treated was then washed well with water to obtain a bioprosthetic valve of the present invention. The resulting bioprosthetic valve was substituted for the aortic valve of a dog, and no calcification was observed on the valve after three months.

### Example 3

The aortic valve of a dog was washed with a saline solution and then immersed in 0.01% aqueous ficin for 24 hours to remove proteins other than collagen. The aortic valve was then washed well with water. Thereafter the aortic valve was allowed to react by immersing in a solution containing 2g of Denacol EX-314®, 0.1g of 2,4,6 - tris (dimethylaminomethyl) phenol as a catalyst and 0.07g of salicylic acid as a reaction accelerator in 100ml of 0.9% aqueous NaCl (pH9.5) at 20°C for 24 hours.

The aortic valve so treated was washed thoroughly with water to obtain a bioprosthetic valve of the present invention, which was stored by immersion in 70% ethanol. This bioprosthetic valve was washed with a physiological sodium chloride solution, and was then implanted into the aortic valve of a dog. The implanted valve was kept open and subject to no calcification after 6 months.

## Claims

1. A bioprosthetic valve comprising a biological tissue derived from a mammal containing crosslinked collagen in which the crosslinked collagen consists essentially of collagen which has been crosslinked with a polyepoxy compound, wherein the polyepoxy compound is selected from glycerol diglycidyl ether, diglycerol triglycidyl ether, diglycerol tetraglycidyl ether and ethylene glycol glycidyl ether.

2. A bioprosthetic valve according to claim 1, wherein the biological tissue comprises a valve of an animal.

3. A bioprosthetic valve according to claim 2, wherein the valve of the animal is selected from porcine aortic valve, ovine aortic valve, bovine aortic valve, mitral valve, tricupsid valve, pulmonary valve or caprine aortic valve.

4. A bioprosthetic valve according to any of claims 1 to 3, wherein the valve has a valve cusp which has been formed of a membrane-like tissue.

5. A bioprosthetic valve according to claim 4, wherein the membrane-like tissue is selected from pericardium, dura mater, amnion or fascia.

6. A bioprosthetic valve according to any of claims 1 to 5, wherein the collagen has been crosslinked to an extent of from 5 to 90% with respect to an ε-amino group.

7. A bioprosthetic valve according to claim 6, wherein the collagen has been crosslinked to an extent of from 10 to 60% with respect to an ε-amino group.

8. A bioprosthetic valve as claimed in any of the preceding claims, which is prepared by immersing the biological tissue in a solution of the polyepoxy compound.

## Patentansprüche

1. Bioprosthetisches Ventil, umfassend ein von einem Säugetier abgeleitetes biologisches Gewebe, das vernetztes Kollagen enthält, in dem das vernetzte Kollagen im wesentlichen aus Kollagen besteht, das mit einer Polyepoxidverbindung vernetzt wurde, wobei die Polyepoxidverbindung ausgewählt ist aus Glycerindiglycidether, Diglycerintriglycidether, Diglycerintetraglycidether und Ethylenglycolglycidether.

2. Bioprosthetisches Ventil nach Anspruch 1, dadurch gekennzeichnet, daß das biologische Gewebe ein Ventil eines Tiers umfaßt.

3. Bioprosthetisches Ventil nach Anspruch 2, dadurch gekennzeichnet, daß das Ventil des Tiers ausgewählt ist aus Schweineaortenventil, Schafsaortenventil, Rinderaortenventil, Mitralventil, Trikuspidalklappe, Lungenventil oder Ziegenaortenventil.

4. Bioprosthetisches Ventil nach einem der Ansprüche 1 bis 3, dadurch gekennzeichnet, daß das Ventil eine Ventilklappe besitzt, die aus einem membranähnlichen Gewebe ausgebildet wurde.

5. Bioprosthetisches Ventil nach Anspruch 4, dadurch gekennzeichnet, daß das membranähnliche Gewebe ausgewählt ist aus Perikardium, Dura Mater, Amnion oder Fascie.

6. Bioprosthetisches Ventil nach einem der Ansprüche 1 bis 5, dadurch gekennzeichnet, daß das Kollagen auf ein Ausmaß von 5 bis 90 %, bezogen auf eine ε-Aminogruppe, vernetzt wurde.

7. Bioprosthetisches Ventil nach Anspruch 6, dadurch gekennzeichnet, daß das Kollagen auf ein Ausmaß von 10 bis 60 %, bezogen auf eine ε-Aminogruppe, vernetzt wurde.

8. Bioprosthetisches Ventil nach einem der vorhergehenden Ansprüche, hergestellt durch Eintauchen des biologischen Gewebes in eine Losung der Polyepoxidverbindung.

## Revendications

1. Bioprothèse valvulaire comprenant un tissu biologique provenant d'un mammifère contenant du collagène réticulé, dans laquelle le collagène réticulé consiste essentiellement en collagène ayant été réticulé par un polyépoxyde, le polyépoxyde étant choisi parmi l'éther diglycidylique de glycérol, l'éther triglycidylique de diglycérol, l'éther tétraglycidylique de diglycérol et l'éther glycidylique d'éthylène-glycol.

2. Bioprothèse valvulaire selon la revendication 1, dans laquelle le tissu biologique comprend une valve d'un animal.

3. Bioprothèse valvulaire selon la revendication 2, dans laquelle la valve de l'animal est choisie parmi une valve aortique porcine, une valve aortique ovine, une valve aortique bovine, une valve mitrale, une valve tricuspide, une valve pulmonaire ou une valve aortique caprine.

4. Bioprothèse valvulaire selon l'une quelconque des revendications 1 à 3, dans laquelle la valve comporte une cuspide valvulaire qui a été formée à partir d'un tissu membranaire.

5. Bioprothèse valvulaire selon la revendication 4, dans laquelle le tissu membranaire est choisi parmi le péricarde, la dure-mère, l'amnios ou l'aponévrose.

6. Bioprothèse valvulaire selon l'une quelconque des revendications 1 à 5, dans laquelle le collagène a été réticulé à un degré de 5 à 90 % par rapport aux groupes ε-amino.

7. Bioprothèse valvulaire selon la revendication 6, dans laquelle le collagène a été réticulé à un degré de 10 à 60 % par rapport aux groupes ε-amino.

8. Bioprothèse valvulaire telle que revendiquée dans l'une quelconque des revendications précédentes, qui est préparée en plongeant le tissu biologique dans une solution du polyépoxyde.
